(19) Europäisches Patentamt
European Patent Office
Office européen des brevets

(11) Numéro de publication: **0 003 461**
**B1**

(12) # FASCICULE DE BREVET EUROPEEN

(45) Date de publication du fascicule de brevet: **29.04.81**

(51) Int. Cl.[3]: **C 07 C 103/44,** C 07 C 93/14, A 61 K 31/215

(21) Numéro de dépôt: **79400048.9**

(22) Date de dépôt: **24.01.79**

(54) **Amino alcools dérivés de l'ortho-hydroxy cinnamate de méthyle, leur procédé de préparation et médicaments les contenant.**

(30) Priorité: **25.01.78 FR 7801964**

(43) Date de publication de la demande: **08.08.79 Bulletin 79/16**

(45) Mention de la délivrance du brevet: **29.04.81 Bulletin 81/17**

(84) Etats contractants désignés: **BE CH DE FR GB IT LU**

(56) Documents cités: **FR-A-2 353 520**

(73) Titulaire: **LIPHA, LYONNAISE INDUSTRIELLE PHARMACEUTIQUE, 115, avenue Lacassagne B.P. No 106 Lyon R.P., F-69212 Lyon Cedex 1 (FR)**

(72) Inventeur: **Briet, Philippe, 216, avenue Félix Faure, F-69003 Lyon (FR)**
Inventeur: **Depin, Jean-Claude, 113, Cours Gambetta, F-69003 Lyon (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 003 461 B1

BUNDESDRUCKEREI BERLIN

Amino alcools dérivés de l'ortho-hydroxy cinnamate de méthyle, leur procédé de préparation et médicaments les contenant

La présente invention conerne des aminoalcools dérivés de l'ortho hydroxy cinnamate de méthyle.

Par la demande de brevet français 2.353.520, on connait des dérivés des hydroxy cinnamate d'éthyle et de terbutyle tels les chlorhydrates de [(diméthoxy-3,4 phényléthylamino)-3 hydroxy-2 propyloxy]-2 cinnamate d'éthyle et d'ortho (diméthoxy-3',4' phényléthylamino-3 hydroxy-2 propoxy) cinnamate de terbutyle, présentés comme des médicaments ayant une activité $\beta$-adrénolytique, $\beta_1$-adrénolytique et/ou hypotensive et/ou anti-arythmique.

On a trouvé les composés représentés par la formule

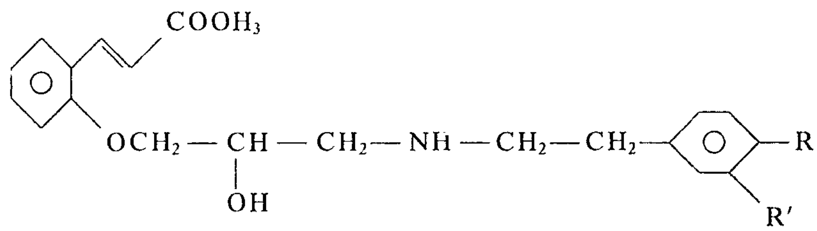

dans laquelle R et R' sont le radical méthoxy, et R est le radical acétamido quand R' est l'hydrogène.

L'invention concerne plus précisément le trans ortho [(3-4 diméthoxyphénéthyl) amino-3 hydroxy-2 propoxy] cinnamate de méthyle (I) et le trans ortho [(acétamido -4 phénéthyl) amino-3 hydroxy-2 propoxy] cinnamate de méthyle (II).

Pour préparer les nouveaux composés on réalise la condensation de l'ortho (époxy-2,3 propoxy) trans cinnamate de méthyle avec l'homovératrylamine pour obtenir le composé (I) ou avec l'acétamido-4 phénéthylamine pour obtenir (II).

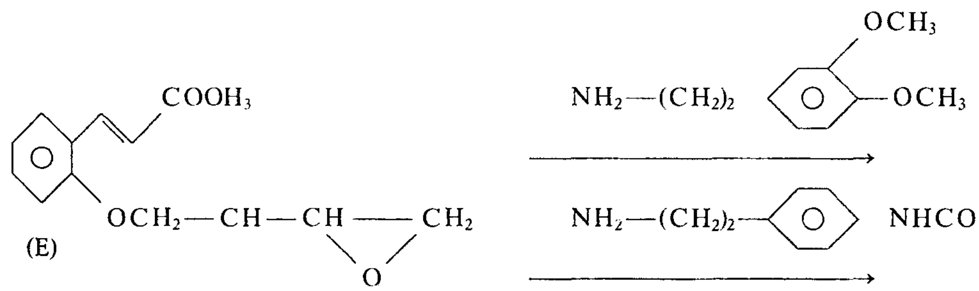

(E) (I) (II)

Selon un mode préférentiel, on réalise cette condensation à chaud, à reflux dans un solvant adéquat tel que l'isopropanol.

L'acétamido-4 phénéthylamine est obtenue par hydrogénation catalytique classique de l'acétamido-4 phényl acétonitrile. En tant que produit intermédiaire nouveau, elle fait partie de l'invention.

Les composés de l'invention possèdent un carbone assymétrique et de ce fait peuvent être dédoublés suivant des techniques bien connues des spécialistes notamment par cristallisation des sels obtenus avec un acide organique optiquement actif.

Les formes optiquement actives obtenues font évidemment partie de l'invention.

Les nouveaux composés possèdent une puissante activité de blocage des récepteurs bêta-sympathiques dans les tissus cardiaques, comparée à l'action dans les autres tissus. Ils marquent de ce fait un perfectionnement important par rapport aux composés de l'art antérieur.

Les deux composés de la demande de brevet français 2.353.520 sont des $\beta$- bloquants cardiosélectifs, mais il s'avère que le composé dérivé de l'orthohydroxy cinnamate de méthyle est le plus actif, très significativement moins toxique, de plus non cardiotoxique.

Dans l'interaction des drogues avec les récepteurs bêta; il est connuet admis que les récepteurs bêta sont de deux types: bêta-1 et bêta-2. (Lands et Coll. NATURE 214, 597) (1967).

Les récepteurs bêta-1 interviennent dans la lipolyse et la stimulation cardiaque alors que les bêta-2 interviennent fans la broncho-dilatation et la vasodépression.

Il apparaît immédiatement tout l'intérêt qu'il y a à posséder des substances qui soient antagonistes des récepteurs bêta-1 et qui n'affectent que peu ou pas les récepteurs bêta-2.

En effect, pour de puissants agents bloquants à la fois des récepteurs bêta-1 et bêta-2 un effet bénéfique sur le coer (effet $\beta_1$) peut s'accompagner d'une bronchoconstriction gênante (effet $\beta_2$).

Il en va autrement pour les composés de l'invention qui font partie de cette catégorie de composés dits cardiosélectifs. Ils sont de ce fait très utiles en médecine humaine, en particulier dans le traitement de l'angor, de la tachyarythmie et de l'hypertension artérielle.

Il a été mis en évidence l'action sur les récepteurs bêta et observé la sélectivité bêta-1 par l'étude de la tension artérielle et du rythme cardiaque du chien anesthésié au pentobarbital et bivagotomisé recevant des injections régulièrement espacées d'isoprénaline (0,4 µg/kg/I. V.).

L'hypotension provoquée par l'isoprénaline est due à une stimulation des récepteurs bêta-2 qui entraîne une vasodilatation. La tachycardie produite par l'isoprénaline est due à une stimulation des récepteurs bêta-1 cardiaques qui entraîne une accélération de son rythme de contraction.

Dans l'inhibition de la tachycardie à l'isoprénaline visualisant un effet de blocage des récepteurs bêta-1, on a déterminé la dose efficace 50 en mg/kg/I. V. (DE 50) pour le Practolol ou 4' - [2-hydroxy-3 - (isopropylamino) propoxy] acétanilide, le propanol ou chlorhydrate d'isopropylamino-1 (naphtoxy-1')-3 propanol-2, le LM 748 ou chlorhydrate de l'ortho (isopropylamino 3 - hydroxy-2 propoxy) trans cinnamate de méthyle et pour deux des composés de la demande.

On a trouvé les valeurs indiqués ci-dessous:

| | |
|---|---|
| Practolol | 0,61 |
| Propranolol | 0,082 |
| LM 748 | 0,014 |
| Composé exemple 1 | 0,048 |
| Composé exemple 2 | 0,085 |

Dans l'inhibition de l'hypotension à l'isoprénaline visualisant un blocage des récepteurs bêta-2 on a déterminé la dose efficace 50 en mg/kg/I. V. (DE 50) pour le Propranolol, le LM 748 et les deux composés de la demande.

On a trouvé pour le Propranolol une DE de 0,036 et pour le LM 748 de 0,01. Par contre, pour les deux composés de la demande, l'action de blocage sur les récepteurs bêta-2 est trop faible pour (u'il soit possible de déterminer valablement une dose efficace 50.

Parmi les observations recueillies en essai clinique, avex administration de comprimés titrés à 200 mg de principe actif, le chlorhydrate de l'ortho [(diméthoxy-3,4 phénéthylamino) -3 hydroxy-2 propoxy] trans cinnamate de méthyle, on peut rapporter les trois suivantes:

### Observation N° 1

Monsieur NOV ....., 64 ans présente un état de mal angineux chronique avec huit crises par 24 heures. Le traitement consiste en un repos et la prise d'un comprimé à 200 mg du composé exemple 1 par 24 heures pendant 10 jours. Le résultat est un effet bradycardisant net; le pouls passe de 95 à 60 et une très nette diminution des crises dès le deuxième jours (2 à 3 par 24 heures) est notée. La tolérance est très bonne.

### Observation N° 2

Monsieur CLI ..., 75 ans présente un bloc auriculoventriculaire complet appareillé par pace-maker cardis à un rythme fixe (68/mn) avec parasystolie. A l'auscultation on note un souffle systolique en écharpe. L'électrocardiogramme montre une parasystolie avec complexes spontanés à P. R. long et aspect de bloc gauche et complexe électroentrainés. La radiographie montre une hypertrophie ventriculaire gauche modérée. Le traitement consiste en la prise journalière pendant 10 jours du composé exemple 1 à 200 mg.

Le résultat est bon. Il y a disparition de la parasystolie par ralentissement de rythme spontané. La tolérance est très bonne.

### Observation N° 3

Monsieur HEL ..., 44 ans a une hypertension artérielle depuis 3 ans; les chiffres habituels récents sont 180/110 m, Mg. L'étiologie est inconnue et il n'y a pas de complications. On associe au traitement consistant en la prise le matin d' 1 comprimé à 200 mg, pendant 15 jours, un régime hyposodé.

Le résultat est très bon. Les chiffres tensionnels sont résumés ci-dessous:

| | Avant | Après |
|---|---|---|
| Tension artérielle couché | 165/102 | 140/90 |
| Tension artérielle debout | 160/100 | 140/88 |
| Pouls | 70 | 60 |

La tolérance est bonne sur le plan général et sur le plan local.

Les compositions pharmaceutiques contenant comme principe actif un composé de l'invention, soit à l'état de base, soit à l'état de sel organique ou minéral, sont présentées sous forme de comprimés, tablettes, gélules, dragées, suspensions aqueuses, solution injectable, sirops et analogues.

Les comprimés peuvent éventuellement être rendus gastro-résistants par un laquage avec un dérivé cellulosique.

Il est donné ci-après à titre d'exemple, une formule de comprimé:

| | |
|---|---|
| Principe actif | 200 mg |
| Lactose | 200 mg |
| Amidon | 25 mg |
| Talc | 12 mg |
| Gélatine | 15 mg |
| Acide alginique | 10 mg |
| Fécule de pomme de terre | 15 mg |
| Stéarate de magnésie | 3 mg |

Ces compositions pharmaceutiques contenant comme principe actif un composé de l'invention et un support ou diluant pharmaceutique solide ou liquide physiologiquement acceptable permettent l'administration journalière de doses de principe actif comprises entre environ 20 et 1000 mg.

Il est donné à titre d'exemple la préparation des deux composés objets de l'invention.

## Exemple 1

trans ortho [(3-4 diméthoxyphénéthyl) amino-3 hydroxy-2 propoxy] cinnamate de méthyle

$C_{23}H_{29}NO_6$   P. M. = 415

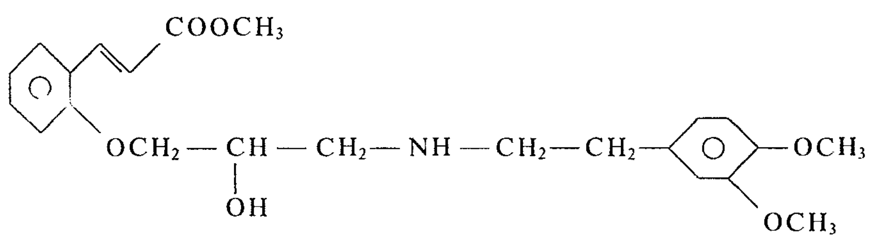

On porte à reflux pendant 6 heures une solution de 135 g (0,58 mole) d'ortho-(époxy-2,3 propoxy) trans cinnamate de méthyle et 90,5 g (0,5 mole) d'homovératrylamine dans 580 ml d'isopropanol. On évapore sous vide les solvants et obtient une résine claire.

Un échantillon cristallisé et recristallisé dans diisopropyléther fond à 79° C. I. R. (KBR)$\nu$—C=O: 1720 $cm^{-1}$ R. M. N. ($CCl_4$) en ppm ($\delta$) par rapport au TMS, pris comme référence interne

— $COOCH_3$ : singulet 3,7

— 2 $OCH_3$ : singulet 3,75

Chlorhydrate: $C_{23}H_{30}ClNO_6$   P. M. = 451,94

On solubilise la résine claire précédemment obtennue dans 750 ml d'éthanol et ajoute goutte à goutte 45 ml d'acide chlorhydrique concentré entre 15 et 20° C. On dilue le milieu avec 750 ml d'éther et place à l'armoire frigorifique. On recueille 114 g d'un solide blanc.

Rendement: (théorique 225 g) = 51%

F° : 157° C (ETOH)

Analyse:

| | Carbone | Hydrogène | Chlore | Azote |
|---|---|---|---|---|
| Calculée % | 61,13 | 6,69 | 7,84 | 3,10 |
| Trouvée % | 61,01 | 6,77 | 7,88 | 3,18 |

Exemple 2

a) acétamido-4 phényléthyl amine $C_{10}H_{14}N_2O$ P. M. : 178,24

On hydrogène sous 150 Kg de pression pendant 6 heures à 80° C:

19 g (0,109 mole) d'acétamido-4 phénylacétonitrile dans 86 ml d'éthanol en présence de 8,6 g de nickel de Raney.

On filtre le catalyseur et évapore à sec. Les cristaux résiduels sont purifiés par recristallisation dans 50 ml d'acétate d'éthyle.

On obtient 14,5 g (Rendement 74%) d'un solide blanc qui fond à 118° C.

R. M. N. (DMSO) déplacement en ppm (δ) par rapport au TMS pris comme référence interne:

1 massif d'aire 2 étalé de 0,8 à 1,4 ($-NH_2$),

1 singulet d'aire 3 à 2 ($CH_3-CO$),

1 massif d'aire 4 étalé de 2,3 à 3,1 ($-CH_2-CH_2-$),

1 massif d'aire 4 étalé de 6,7 à 7,6

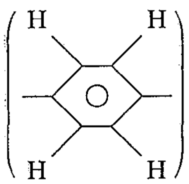

1 massif d'aire 1 étalé de 8,95 à 9,45 ($CO-NH$)

b) trans ortho [(acétamido-4 phénéthyl) amino-3 hydroxy-2 propoxy] cinnamate de méthyle

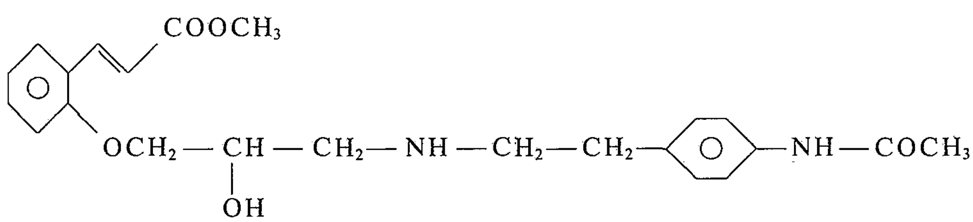

(II)

On porte à reflux 6 heures: 9,4 g (0,04 mole) d'ortho (époxy-2,3 propoxy) trans cinnamate de méthyle, 6,8 g (0,038 mole) d'acétamido-4 phényléthylamine dans 40 ml d'isopropanol. On évapore sous vide le solvant et disperse sous acétone le solide obtenu. On obtient 7,9 g d'un solide blanc qui fond à 150° C. Rendement 50% R. M. N. (DMSO) Déplacement en ppm (δ) par rapport au TMS pris comme référence interne:

1 singulet d'aire 3 à 2,1 ($-CO-CH_3$),

1 massif d'aire 14 étalé de 2,4 à 4,4 où culmine 1 singulet à 3,7 ($COOCH_3$),

1 massif d'aire 10 étalé de 6,3 à 8,2 (8 aromatiques et 2 H cinnamiques trans (J = 16 cps),

1 singulet d'aire 1 à 9,6 ($CO-NH$)

Oxalate Acide: $C_{25}H_{30}N_2O_9$ P. M.: 502,53

F° = 151 — 152° C (acétone-eau)

Analyse:

| | Carbone | Hydrogène | Azote |
|---|---|---|---|
| Calculée % | 59,75 | 6,02 | 5,57 |
| Trouvée % | 59,67 | 6,07 | 5,59 |

## Revendications

1. Amino-alcools dérivés de l'ortho-hydroxycinnamate de méthyle représentés par la formule

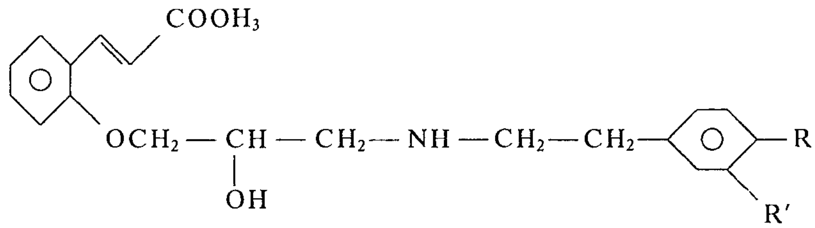

dans laqzelle R et R' sont le radical méthoxy et R est le radical acétamido quand R' est l'hydrogène, à l'état de base, de sel organique ou minéral.

2. Amino-alcool selon la revendication 1, le trans ortho [(3,4 - diméthoxyphénéthyl) amino-3 hydroxy-2 propoxy] cinnamate de méthyle.

3. Amino-alcool selon la revendication 1, le trans ortho[(acétamido-4 phénéthyl) amino-3 hydroxy-2 propoxy] cinnamate de méthyle.

4. Procédé de préparation des composés selon la revendication 1 ou 2, caractérisé en ce que l'on condense l'ortho (époxy-2,3 propoxy) trans cinnamate de méthyle avec une amine de formule

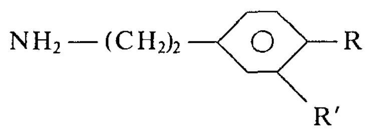

dans laquelle R et R' ont les mêmes significations que précédemment.

5. A titre de produit intermédiaire dans la préparation du composé selon la revendication 3, l'acétamido-4 phénéthylamine de formule

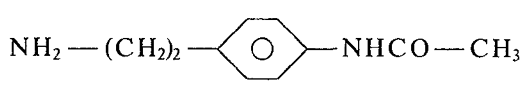

6. Médicament contenant comme principe actif un amino alcool dérivé de l'ortho-hydroxycinnamate de méthyle représenté par la formule

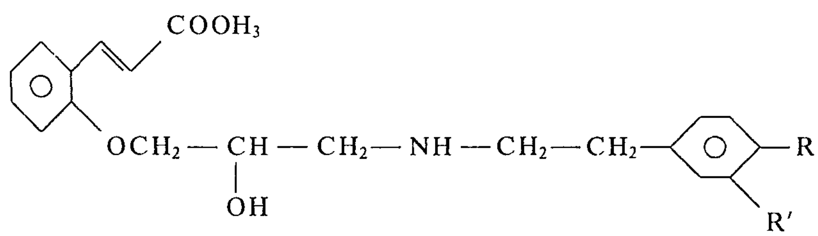

dans laquelle R et R' sont le radical méthoxy et R est le radical acétamido quand R' est l'hydrogène, à l'état de base, de sel organique ou minéral.

7. Composition pharmaceutique caractérisée en ce qu'elle contient comme principe actif un composé selon la revendication 6, associé à un support ou diluant pharmaceutique solide ou liquide physiologiquement acceptable.

8. Composition pharmaceutique selon la revendication 7, caractérisée en ce qu'elle permet l'administration de doses de principe actif comprises entre 20 et 1000 mg.

**Patentansprüche**

1. Sich von ortho-Hydroxyzimtsäuremethylester herleitende Aminoalkohole der allgemeinen Formel

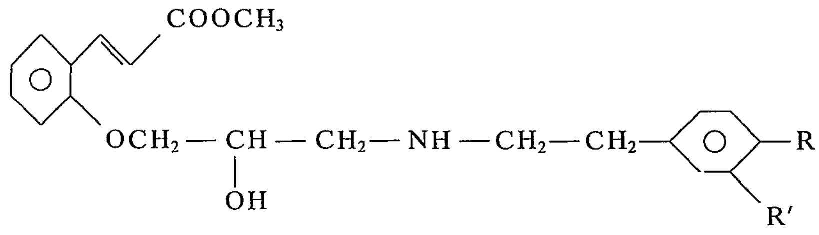

worin R und R′ der Methoxyrest sind und R der Acetamidorest ist, wenn R′ ein Wasserstoffatom ist, in der Form der freien Base oder eines Salzes einer organischen Säure oder Mineralsäure.

2. Aminoalkohol nach Anspruch 1, der trans-ortho-[3-(3,4-Dimethoxyphenäthyl)-amino-2-hydroxy-propoxy]-zimtsäuremethylester ist.

3. Aminoalkohol nach Anspruch 1, der trans-ortho-[3-(4-Acetaminophenäthyl)-amino-2-hydroxy-propoxy]-zimtsäuremethylester ist.

4. Verfahren zur Herstellung von Verbindungen nach Anspruch 1 oder 2, dadurch gekennzeichnet, daß man den ortho-(2,3-Epoxy-propoxy)-trans-zimtsäuremethylester mit einem Amin der Formel

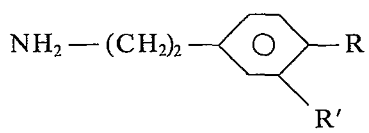

worin R und R′ die gleiche Bedeutung wie oben haben, kondensiert.

5. Als Zwischenprodukt für die Herstellung der Verbindung nach Anspruch 3 das 4-Acetamido-phenäthylamin der Formel

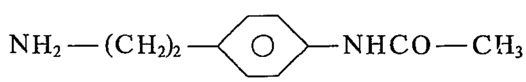

6. Arzneimittel enthaltend als Wirkstoff einen sich von dem ortho-Hydroxyzimtsäuremethylester herleitenden Aminoalkohol der Formel

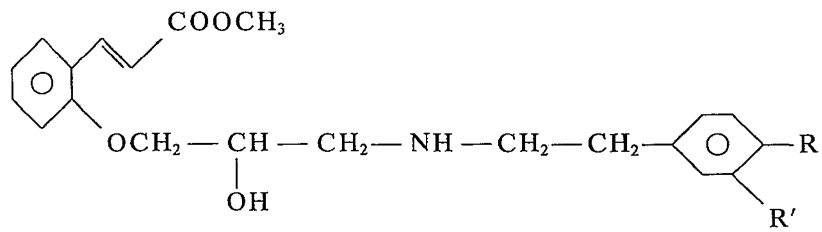

worin R und R′ der Methoxyrest sind und R der Acetamidorest ist, wenn R′ ein Wasserstoffatom ist, in der Form der freien Base oder eines Salzes einer organischen Säure oder Mineralsäure.

7. Pharmazeutisches Präparat, dadurch gekennzeichnet, daß es als Wirkstoff eine Verbindung nach Anspruch 6 zusammen mit einem festen oder flüssigen, physiologisch verträglichen pharmazeutischen Trägermaterial oder Verdünnungsmittel enthält.

8. Pharmazeutisches Präparat nach Anspruch 7, dadurch gekennzeichnet, daß es die Verabreichung von Dosen des Wirkstoffes zwischen 20 und 1000 mg erlaubt.

**Claims**

1. Amino alcohols derived from ortho methyl hydroxy cinnamate having the formula

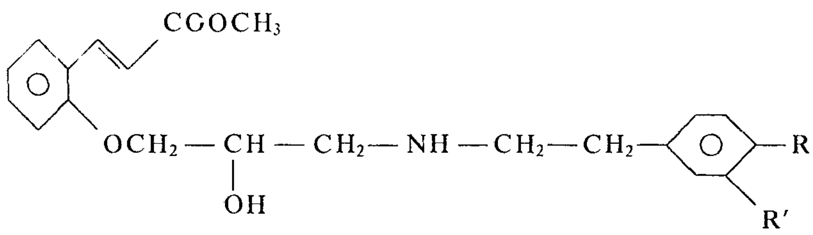

wherein R and R′ are the methoxy radical and R′ is the acetamido radical, if R′ is hydrogen, in the form of the free base or a salt of an organic or mineral acid.

2. Amino alcohol according to claim 1, the trans ortho-[3-(3,4-dimethoxy phenethyl)-amino-2-hydroxy-propoxy] methyl cinnamate.

3. Amino alcohol according to claim 1, the trans ortho-[3-(4 acetamido-phenethyl)-amino-2-hydroxy-propoxy] methyl cinnamate.

4. Process for the production of the compounds according to claim 1 or 2, characterized in that the ortho (2,3-epoxy-propoxy)trans methylcinnamate is condensed with an amine having the formula

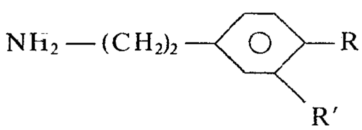

wherein R and R′ have the same meaning as above.

5. As intermediate product in the preparation of a compound according to claim 3, the 4-acetamido-phenethylamine having the formula

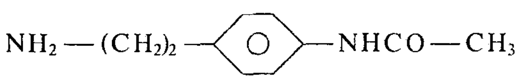

6. Medicament containing as active component an amino alcohol derived from the ortho methylhydroxycinnamate having the formula

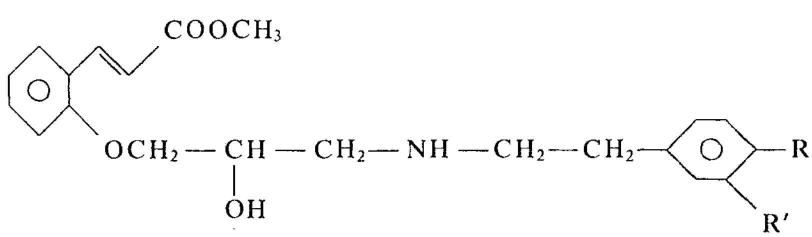

7. Pharmaceutical composition, characterized in that it contains as active component a compound according to claim 6 combined with a solid or liquid, physiologically acceptable pharmaceutical support or diluent.

8. Pharmaceutical composition according to claim 7, characterized in that it permits the administration of doses of the active component between 20 and 1000 mgs.